# EUROPEAN PATENT SPECIFICATION

(11) **EP 1 246 827 B1**
(45) Date of publication and mention of the grant of the patent: **13.04.2005**
(21) Application number: 00983422.7
(22) Date of filing: 22.12.2000
(51) Int. Cl.: C07D 495/04, A61K 31/55, A61P 25/18

(54) **NEW POLYMORPHIC FORMS OF OLANZAPINE**
NEUE POLYMORPHE FORMEN VON OLANZAPIN
NOUVELLES FORMES POLYMORPHES D'OLANZAPINE

(30) Priority: 28.12.1999 IN BO097799; 31.03.2000 US 540749
(43) Date of publication of application: 09.10.2002
(73) Proprietor: CIPLA LIMITED, Bombay-400008, Maharashtra (IN)
(72) Inventor: HAMIED, Yusuf Khwaja, Off Bhulabhai Desai Rd, Mumbai 400026 (IN); KANKAN, Rajendra Narayanrao, A 3/5, NBD Society, Mumbai 400 084, Maharashtra (IN); RAO, Dharmaraj Ramachandra, Thane 400 601, Maharashtra (IN)
(74) Representative: Wain, Christopher Paul
(86) International application number: PCT/GB2000/004982
(87) International publication number: WO 2001/047933

(56) References cited:
- EP-A- 0 733 635
- EP-A- 0 831 097
- WO-A-00/18408
- US-A- 5 919 485
- HOUBEN-WEYL: "Methoden der organischen Chemie, Band I/1, Allgemeine Laboratoriumspraxis I" 1958 , GEORG THIEME VERLAG , STUTTGART XP002163768 page 381 -page 382

## Description

This invention relates to novel forms of 2-methyl-4-(4-methy(-1-piperazinyl)-10H-thieno-[2,3b][1,5] benzodiazepine (Formula A), also known as olanzapine. More specifically, the invention provides novel forms of solvate free olanzapine, methods for preparing the novel forms of olanzapine and pharmaceutical formulations containing the novel forms of olanzapine.

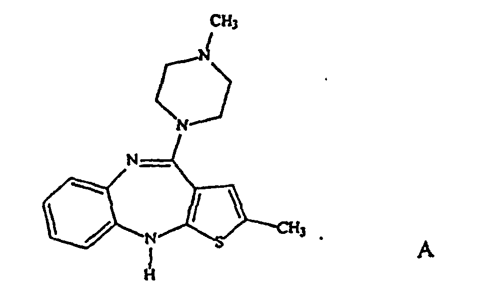

As described in U.S. Patent No. 5,736,541 (hereinafter "the '541 patent"), the synthesis of olanzapine according to the methods described in U.S. Patent No. 5,229,382 produces a metastable, dull colored product referred to in the '541 patent as "Form I." The '541 patent is herein incorporated by reference in its entirety. The '541 patent discloses and claims a more stable polymorphic form of olanzapine, designated as "Form II", a method to produce "Form II" olanzapine, and pharmaceutical compositions containing "Form II" olanzapine. "Form I" and "Form II" olanzapine are characterized in the '541 patent by powder X-ray diffraction. The interplanar spacings (d-spacings) and typical relative intensities (I/I₁) are reported.

U.S. Patent No. 5,703,232 (hereinafter "the '232 patent") claims lower alcohol solvates of olanzapine referred to in the '232 patent as "Form I" and methods for their preparation. The polymorph designated as "Form I" in the '232 patent has the same characteristic interplanar spacing by X-ray diffraction as "Form II" of the '541 patent and should thus be considered the same polymorph. Similarly, the polymorph designated as "Form II" in the '232 patent has the same characteristic interplanar spacing by X-ray diffraction as the polymorph designated as "Form I" in the '541 patent and should thus be considered the same polymorph. As used hereinafter the terms "Form I" and "Form II" refer to the olanzapine products designated as "Form I" and "Form II" in the '541 patent having the interplanar spacings and typical relative intensities shown in Table 1.

The present invention satisfies a need for additional stable, anhydrous and solvate-free polymorphic forms of olanzapine useful in the preparation of pharmaceutical formulations.

Accordingly, the present invention provides olanzapine polymorphs Form III, Form IV and Form V as defined in the claims.

The present invention provides new polymorphic forms of 2-methyl-4-[4-methyl-1-piperazinyl]-10H-thieno[2,3b][1,5] benzodiazepine (olanzapine) designated as "Form III", "Form IV" and "Form V", methods of preparing the new polymorphic forms of olanzapine and pharmaceutical compositions containing them.

The invention produces new substantially pure polymorphs of olanzapine in high yield. The invention further differs from the prior art by requiring only aqueous solvents to prepare the stable polymorphs. The invention also provides an advantage over the prior art by isolating the new olanzapine polymorphs in a solvent free media, thus producing olanzapine free of solvates and having a negligible solvent content.

The invention provides three novel, solvate free forms of olanzapine designated Form III, Form IV and Form V. The novel forms of olanzapine are characterised by their unique x-ray diffraction patterns and infrared spectra.

According to the present invention, there is provided a process for producing a polymorph of olanzapine as defined in the claims, which process comprising dissolving an initial polymorph of olanzapine in aqueous acidic solution; wherein the aqueous acidic solution comprises an organic or inorganic acid; and precipitating a different polymorph of olanzapine by neutralisation; wherein neutralisation is accomplished by the addition of an aqueous or alcoholic solution of a base.

The process for preparing the novel forms of olanzapine involves first dissolving olanzapine in an aqueous organic or inorganic acid, which may for example be acetic acid, formic acid, hydrochloric acid, sulphuric acid, citric acid, fumaric acid or maleic acid; and is preferably hydrochloric acid, sulphuric acid, formic acid or acetic acid. The new form of olanzapine is then precipitated using an aqueous or alcoholic solution of alkali, which may for example by potassium hydroxide, sodium hydroxide or ammonia. The alcoholic solvent may be any mono, di, or polyhydric alcohol, preferably methanol. The olanzapine obtained typically contain less than 5% of other forms of olanzapine and less than 1% of other impurities. The desired form of olanzapine can be obtained by varying the acid or its concentration, and the temperature and pH of precipitation. The acid solution used in preparing the novel forms of olanzapine may contain between about 5% and about 50% acid. Olanzapine is preferably precipitated at a temperature between about 0°C and about 100°C, more preferably between about 0°C and about 35°C and most preferably between about 10°C and about 30°C. The final pH of the solution, after precipitation, is preferably between about 6 and about 12, and more preferably between about 8 and about 11.

The invention also provides pharmaceutical formulations containing as an active ingredient at least one of the novel forms of olanzapine according to the invention or a pharmaceutically acceptable salt thereof. The invention further provides a method of treating a psychotic condition, mild anxiety or gastrointestinal conditions by administering an effective amount of at least one of Form III, Form IV or Form V olanzapine or a pharmaceutically acceptable salt thereof to a patient.

The above objectives and advantages of the invention are illustrative, and not exhaustive, of those which can be achieved by the invention and the examples presented herein are non-limiting. Thus, these and other objectives and advantages of the invention will be apparent from the description herein, both as embodied herein and as modified in view of any variations which will be apparent to those skilled in the art.

### BRIEF DESCRIPTION OF THE DRAWINGS

Embodiments of the invention are explained in greater detail by way of the accompanying figures:
Figure 1 is a FT-IR spectrum of Form III olanzapine.
Figure 2 is a FT-IR spectrum of Form IV olanzapine.
Figure 3 is a FT-IR spectrum of Form V olanzapine.
Figure 4 is the X-ray diffraction pattern obtained for Form III olanzapine.
Figure 5 is the X-ray diffraction pattern obtained for Form IV olanzapine.
Figure 6 is the X-ray diffraction pattern obtained for Form V olanzapine.

### DETAILED DESCRIPTION OF THE INVENTION

The process of the present invention consists of dissolving olanzapine in aqueous acid and precipitating olanzapine from the resultant salt solution using an aqueous or alcoholic solution of alkali. Alcoholic solutions include any mono-, di- or polyhydric alcohol. A methanolic solution is particularly preferred. The acids to be used in the present invention may be any suitable organic or inorganic acid, for example, hydrochloric acid, sulfuric acid, acetic acid, formic acid, citric acid, fumaric acid, and maleic acid. Other acids include oxalic acid, lactic acid, methane sulphonic acid and hydrobromic acid. Preferred acids are hydrochloric acid, sulfuric acid, acetic acid and formic acid. The concentration of acid may range from 5% to 50%.

Either Form I or Form II olanzapine may be used as a starting material in the invention. The preferred olanzapine used in preparing the novel polymorphs of the invention is Form I olanzapine obtained by the method described in U.S. Patent No. 5,229,382, which is herein incorporated by reference in its entirety.

The Form I or Form II olanzapine is mixed with the selected acid and stirred at a suitable temperature until dissolved completely. The solution is then neutralised using a base selected from for example aqueous or alcoholic sodium hydroxide, aqueous or alcoholic potassium hydroxide or aqueous ammonia. Other bases include organic bases such as aqueous monomethyl amine, aqueous dimethylamine and pyridine. Sodium and potassium carbonates and bicarbonates may also be used, although they are not generally preferred owing to effervescence during neutralisation. The alcohol solvent may be any mono, di, or polyhydric alcohol. Methanol is a preferred alcoholic solvent, as is ethanol and isopropanol.

The term "neutralisation" is to be understood in its broadest sense, meaning the addition of base to ensure complete neutralisation of the acid.

The temperature of precipitation is preferably between about 0°C and about 100°C, more preferably between about 0°C and about 35°C and most preferably between about 10°C and about 30°C. During precipitation, the pH of the precipitate is preferably adjusted to be between about 6 and about 12, and more preferably between about 8 and about 11. The novel polymorphs of the invention are obtained in substantially pure form. The term "substantially pure" as used herein means that the polymorphs contain less that 5% of other forms of olanzapine and less than 1% of other impurities, water or solvates.

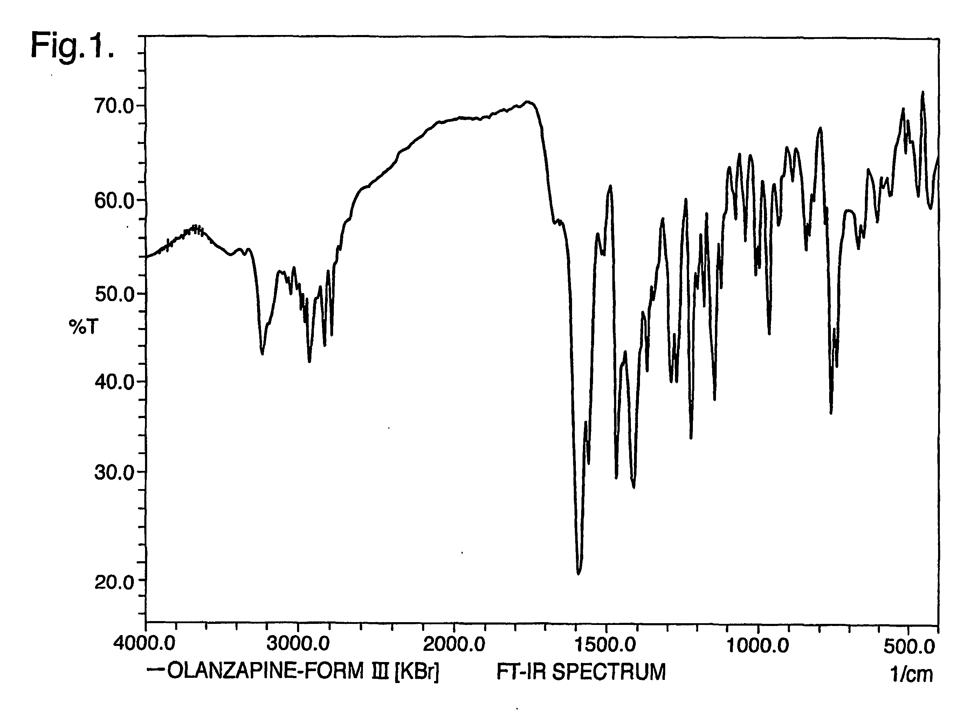

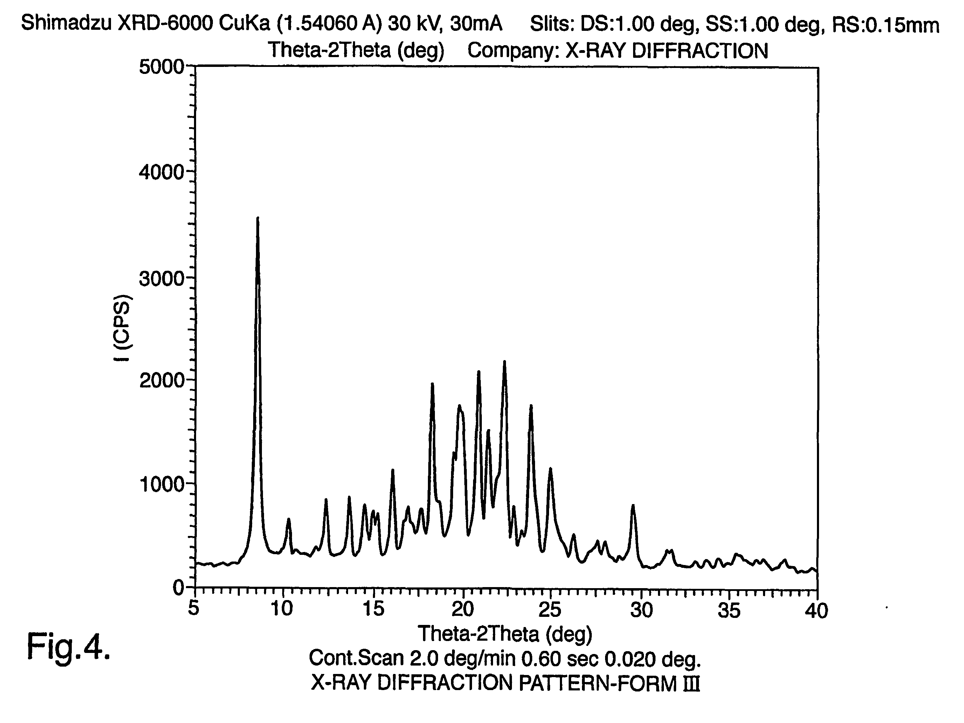

The novel polymorphs of the invention have been characterized by powder x-ray diffraction (XRD) patterns obtained using a Shimadzu X-ray diffractometer XRD-6000, equipped with a wide range goniometer and using copper Kα radiation as set forth in Figures 4-6. The interplanar spacings (in Angstroms) and typical relative intensities (I/I₁) sufficient to identity Forms III, IV, and V olanzapine according to the invention are set forth in Table 3. The complete set of interplanar spacings and relative intensities for Forms III, IV, and V olanzapine are set forth in Table 5. The novel polymorphs were further characterized by infrared (IR) spectroscopy obtained in a KBr disk using a Shimadzu FT-IR 8201 PC system as set forth in Figures 1-3. The IR absorbances (in wavenumbers, cm⁻¹) sufficient to identify Forms III, IV, and V olanzapine are set forth in Table 2. The complete set of IR absorbances for Forms III, IV, and V olanzapine are set forth in Table 4.

Form III olanzapine may be prepared by dissolving Form I or Form II olanzapine in about 50% aqueous acetic acid and precipitating the compound using about 15% aqueous ammonia to a final pH of about 8. Alternatively, Form III olanzapine may be obtained by dissolving Form I or Form II olanzapine in about 33% aqueous acetic acid and precipitating the compound using about 50% aqueous sodium hydroxide to a pH of about 10. Form III olanzapine is characterized by the infrared (IR) spectrum of Figure I and by the X-ray diffraction pattern (XRD) of Figure 4. The IR absorbances and XRD peaks sufficient to identify Form III olanzapine are contained in Tables 2 and 3, respectively. The complete set of IR absorbances and XRD peaks for Form III olanzapine are provided in Tables 4 and 5, respectively.

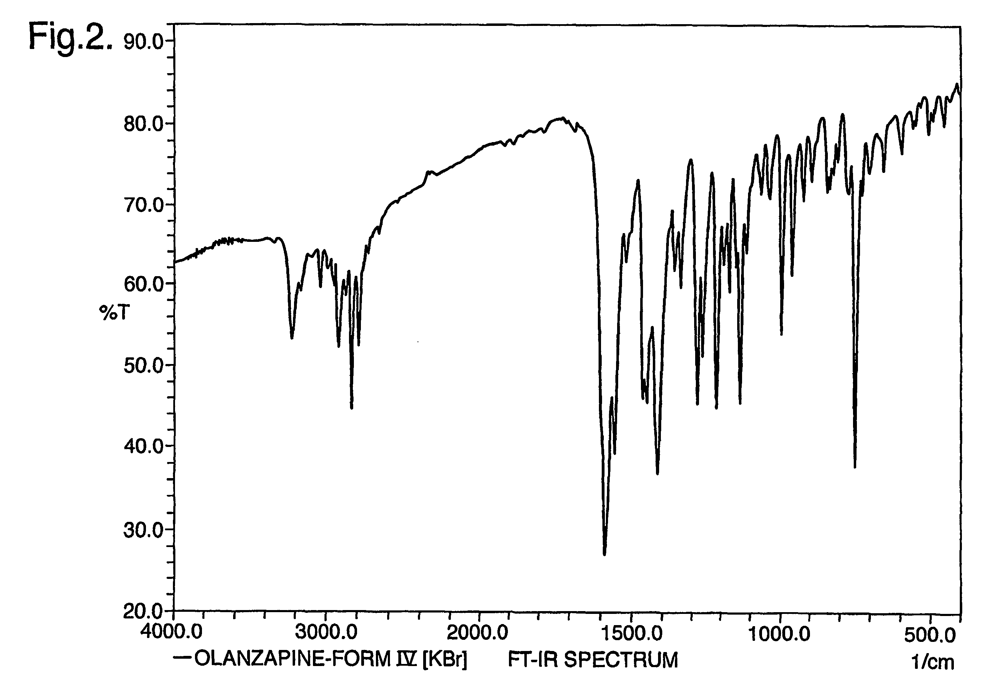

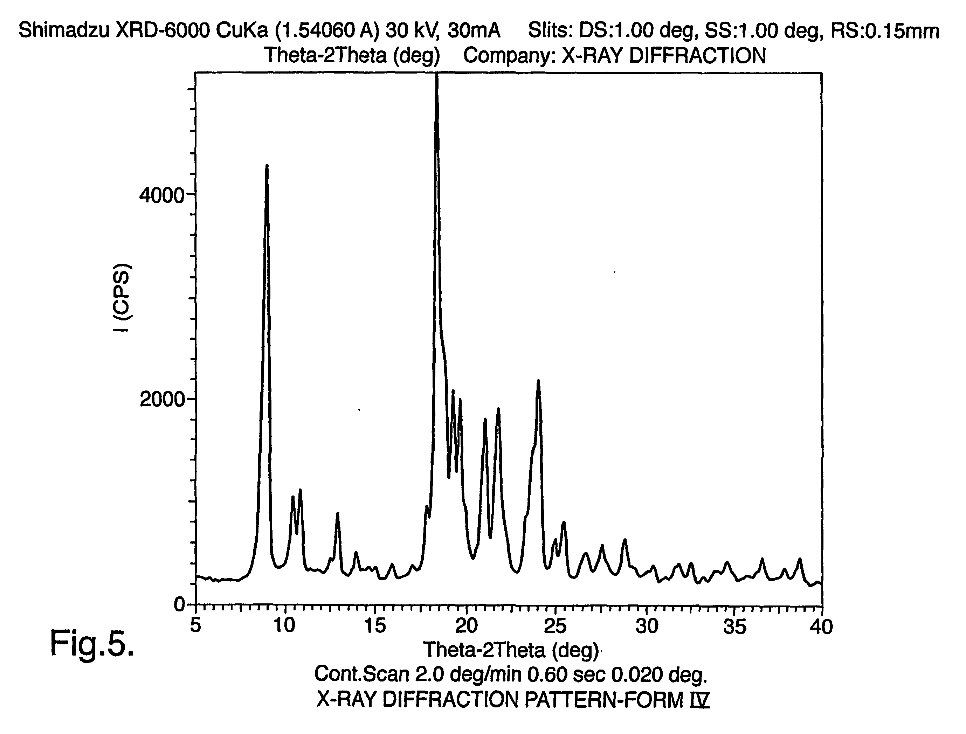

Form IV olanzapine may be prepared by dissolving Form I or Form II olanzapine in about 38% aqueous formic acid and precipitating the compound using about 10% methanolic sodium hydroxide to a final pH of about 8. Alternatively, Form IV olanzapine may be prepared by dissolving Form I or Form II olanzapine in about 43% aqueous acetic acid and precipitating the compound using about 25% ammonia to a final pH of about 10. Form IV olanzapine is characterized by the IR spectrum of Figure 2 and by the.XRD of Figure 5. The IR absorbances and XRD peaks sufficient to identify Form IV olanzapine are contained in Tables 2 and 3, respectively. The complete set of IR absorbances and XRD peaks for Form IV olanzapine are provided in Tables 4 and 5, respectively.

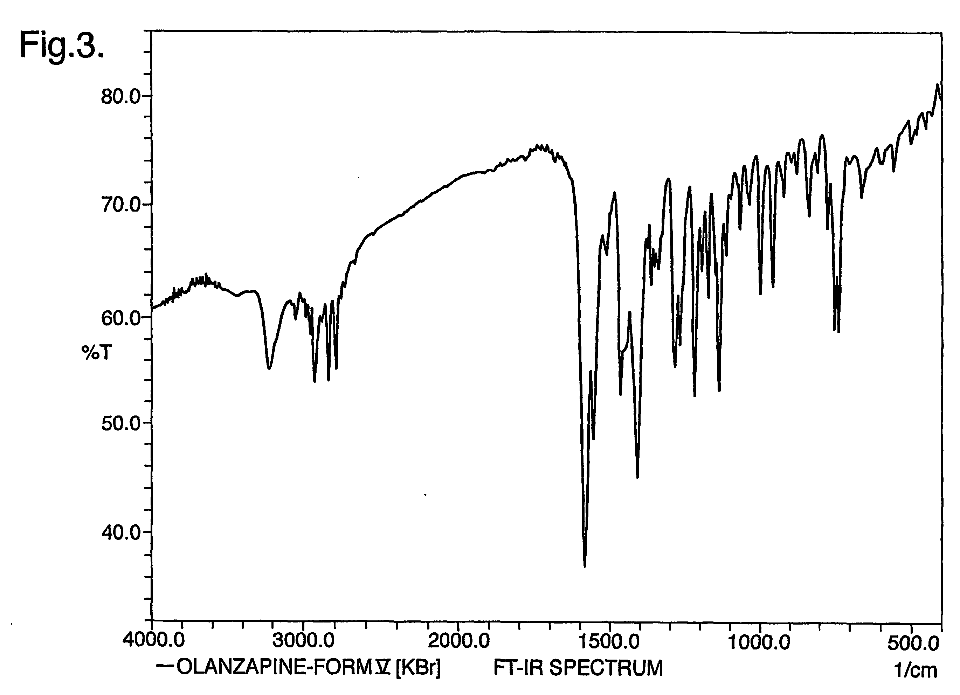

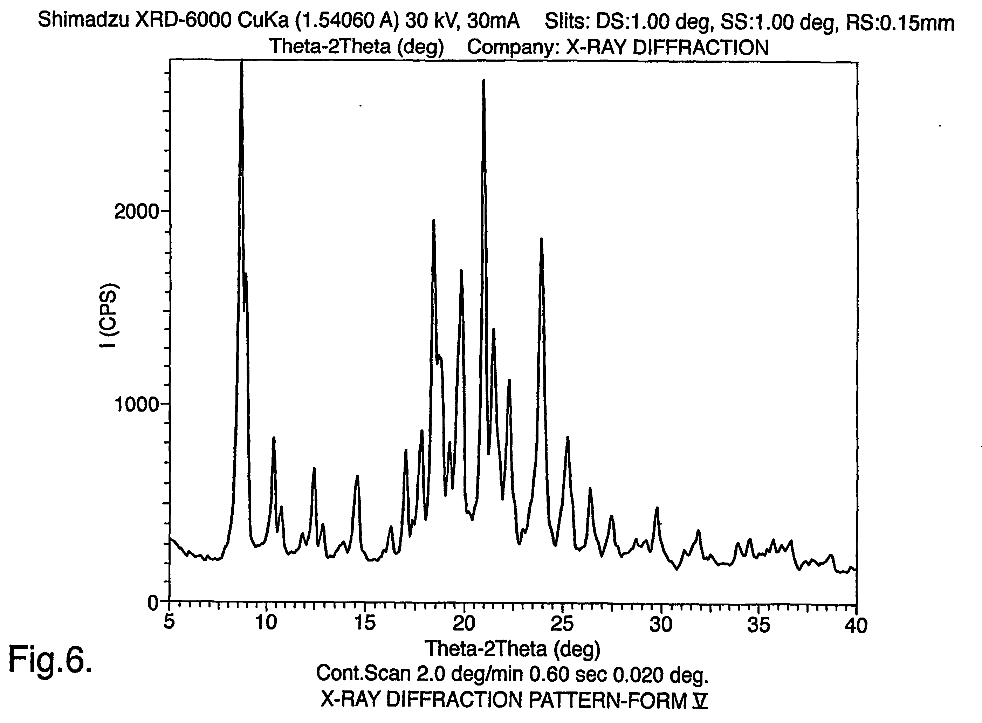

Form V olanzapine may be prepared by dissolving Form I or Form II olanzapine in about 10% aqueous hydrochloric acid and precipitating the compound using about 10% aqueous sodium hydroxide to a final pH of about 8.5. Alternatively, Form V olanzapine may be prepared by dissolving Form I or Form II olanzapine in about 40% aqueous acetic acid and precipitating the compound using about 50% aqueous sodium hydroxide to a final pH of about 9. Form V olanzapine may also be obtained by dissolving Form I or Form II olanzapine in about 20% formic acid and precipitating the compound using about 25% aqueous ammonia. Also, Form V olanzapine may be prepared by dissolving Form I or Form II olanzapine in about 50% acetic acid and precipitating the compound using about 25% ammonia to a final pH of about 9. Farm V olanzapine is characterized by the IR spectrum of Figure 3 and by the XRD of Figure 6. The IR absorbances and XRD peaks sufficient to identify Form V olanzapine are contained in Tables 2 and 3, respectively. The complete set of IR absorbances and XRD peaks for Form V olanzapine are provided in Tables 4 and 5, respectively.

The methods of the invention may be used for the purification of olanzapine, as well as for preparation of the new polymorphic forms. For example, 97% pure (by HPLC) Form I olanzapine may be converted to approximately 99% pure Form III olanzapine (HPLC) by dissolving olanzapine in about 33% aqueous acetic acid and precipitating Form III olanzapine using about 50% aqueous sodium hydroxide to a final pH of about 10.

Olanzapine has been found to have a wide range of therapeutic effects, particularly for the treatment of schizophrenia, schizophrenifocm disorders, psychosis, mild anxiety states and functional bowel disorders. The various disorders which may be treated using olanzapine are described in detail in the '541 patent at column 4, line 62 through column 8, line 55.

Pharmaceutical formulations according to the invention comprise Form III, IV or V olanzapine or a pharmaceutically acceptable salt thereof as an active ingredient together with one or more pharmaceutically acceptable carriers, excipients or diluents. Any conventional technique may be used for the preparation of pharmaceutical formulations according to the invention. Examples of suitable carriers include sugars, starches, gum acacia, calcium phosphate, alginates, tragacanth, gelatin, syrup, methyl cellulose, methyl- and propyl-hydroxy-benzoate, talc, magnesium stearate or mineral oil. The active ingredient may be contained in a formulation that provides quick release, sustained release or delayed release after administration to the patient.

Pharmaceutical compositions may be formulated for transdermal delivery, oral delivery or as a suppository. Formulations may be in the form of capsules, tablets or gels for oral delivery or as a suspension for transermal delivery. Pharmaceutical compositions according to the present invention may preferrably contain 0.25 to 100 mg of active ingredient or, more prefereably, 1 to 30 mg active ingredient, along with a pharmaceutically acceptable carrier.

### Methods

Form I olanzapine used as a starting material was obtained by the method described in U.S. Patent No. 5,229,382. IR-Spectra were obtained in a KBr disk using a Shimadzu FT-IR 8201 PC system. The IR-Spectra obtained for the three polymorphic forms, i.e. Forms III, IV, and V, are shown in Figures 1, 2 and 3, respectively. A summary of wavenumbers sufficient to identify Forms III, IV and V olanzapine is provided in Table 2. Table 4 contains a complete listing of IR absorbances for Forms III, IV and V olanzapine according to the invention. Powder x-ray diffraction patterns were obtained on a Shimadzu X-ray diffractometer XRD-6000, equipped with a wide range goniometer using copper Kα radiation. The powder x-ray diffraction patterns for the three polymorphic forms, i.e. Forms III, IV, and V, are provided in Figures 4, 5 and 6, respectively. The interplanar d-spacings sufficient to identify Forms III, IV and V olanzapine and their relative intensities are set forth in Table 3. The complete set of interplanar d-spacings and relative intensities for Forms III, IV and V olanzapine are provided in Table 5.

### EXAMPLE 1:

Form I olanzapine (10g) was dissolved in a mixture of 30 ml acetic acid and 30 ml water by stirring. Activated charcoal (0.5 g) was added and the contents filtered over celite. The clear solution was maintained at 20°C and 15% aqueous ammonia solution was added over a period of 30 minutes to adjust the pH to 8. The contents were filtered and dried to obtain Form III olanzapine (9.6g), which was characterized by IR and XRD.

### EXAMPLE 2 :

Form I olanzapine (10 g) was dissolved in a mixture of 30 ml acetic acid and 40 ml water and the contents filtered over celite. The clear solution was maintained at 20°C and 30 ml of 25% aqueous ammonia solution was added rapidly in 10 minutes to adjust the pH to about 6. The solids precipitated slowly and the solution was stirred for 30 minutes. A further 30 ml of ammonia solution was added to the mass to obtain a pH of about 10. The contents were further stirred for 1 hour and filtered and dried to obtain Form IV olanzapine (9.4 g), which was charcterized by IR and XRD.

### EXAMPLE 3 :

Form I olanzapine (10 g) was disolved in a mixture of 40 ml acetic acid and 60 ml water and the contents filtered over celite. The clear solution was maintained at 20°C and 50 ml of 50% aqueous sodium hydroxide solution was added rapidly with stirring to obtain a gummy mass. On stirring for a further 30 minutes, a fine suspension was obtained. The pH of the contents was adjusted to about 9 using additional sodium hydroxide solution. The product was recovered by filtration and dried to obain Form V olanzapine (9.4 g), which was characterized by IR and XRD.

### EXAMPLE 4 :

Form I olanzapine (10 g) was dissolved in a mixture of 25 ml formic acid and 40 ml water by stirring. Activated charcoal (0.5 g) was added and the contents were filtered over celite. The clear solution was maintained at 10 to 15°C and neutralized with 10% methanolic sodium hydroxide solution to a pH of 8. The product was recovered by filtration and dried to obtain Form IV olanzapine (9.3 g), which was characterized by IR and XRD.

### EXAMPLE 5 :

Form I olanzapine (10 g) was dissolved in a mixture of 10 ml formic acid and 40 ml water and the solution was filtered over celite. This solution was added slowly to a stirred 25% aqueous ammonia solution (70 ml) to which a few seed crystals of Form V olanzapine were added. The temperature was maintained between 15 to 25°C during the addition. The contents were stirred for 1 hour and filtered and dried to obtain Form V olanzapine (9.4 g), which was characterized by IR and XRD.

### EXAMPLE 6 :

Form I olanzapine (10 g) obtained by the method described in U.S. Patent No. 5,229, 352 and having a purity of 97% (HPLC) was dissolved in a mixture of 30 ml acetic acid and 60 ml water and the solution filtered over celite. The solution was maintained at 20°C with stirring and 50% aqueous sodium hydroxide solution added to adjust the pH to between 6 and 6.2. The solids which precipitated were stirred for 45 minutes and filtered. The wet cake was taken up in water (50 ml) and additional sodium hydroxide added to adjust the pH to 10. The contents were stirred for 1 hour and filtered. The product was dried to obtain >99% pure Form III olanzapine (9.1 g), which was characterized by IR and XRD.

### EXAMPLE 7 :

Form I olanzapine (10 g) was dissolved in 50 ml of 10% hydrochloric acid with stirring. Activated charcoal (0.5 g) was added and the contents filtered over celite. The clear solution was maintained at 15°C and neutralized to a pH of 8.5 with 10% aqueous sodium hydroxide solution. The product was recovered by filtration and dried to obtain Form V olanzapine (9.5 g), which was characterized by IR and XRD.

### EXAMPLE 8:

Form I olanzapine (10 g) was dissolved in a mixture of 30 ml acetic acid and 30 ml water and the contents filtered over celite. This solution was added to 60 ml of a stirred 25% aqueous ammonia solution seeded with a few crystals of Form V olanzapine. The temperature was maintained at 15 to 25°C during the addition of the Form I solution to the aqueous ammonia solution and the pH of the mass was 9 after completion of the addition. After stirring for 1 hour, the product was recovered by filtration and dried to obtain Form V olanzapine which was characterized by IR and XRD.

All of the above Examples can be equally well operated using Form II olanzapine as starting material instead of Form I olanzapine.

Certain representative embodiments of the invention are described in the examples given above. The materials used and the process steps are intended as illustrative of the invention and the invention is not limited to the methods, process steps or any other conditions described in the examples. The examples are non-limiting and may be modified or varied, and elements added or omitted, without departing from the invention, as appreciated by those skilled in the art.

**TABLE - 1**

| X-RAY DIFFRACTION PEAKS OF FORM I AND FORM II OLANZAPINE SUMMARY OF d-spacing AND I/I₁ INTENSITY RATIO | | | |
|---|---|---|---|
| FORM I | | FORM II | |
| d-spacing | I/I₁ | d-spacing | I/I₁ |
| 9.9463 | 100.00 | 10.2689 | 100.00 |
| 8.5579 | 15.18 | 8.577 | 7.96 |
| 8.2445 | 1.96 | 7.4721 | 1.41 |
| 6.8862 | 14.73 | 7.125 | 6.50 |
| 6.3787 | 4.25 | 6.1459 | 3.12 |
| 6.2439 | 5.21 | 6.071 | 5.12 |
| 5.5895 | 1.10 | 5.4849 | 0.52 |
| 5.3055 | 0.95 | 5.2181 | 6.86 |
| 4.9815 | 6.14 | 5.1251 | 2.47 |
| 4.8333 | 68.37 | 4.9874 | 7.41 |
| 4.7255 | 21.88 | 4.7665 | 4.03 |
| 4.6286 | 3.82 | 4.7158 | 6.80 |
| 4.533 | 17.83 | 4.4787 | 14.72 |
| 4.4624 | 5.02 | 4.3307 | 1.48 |
| 4.2915 | 9.19 | 4.2294 | 23.19 |
| 4.2346 | 18.88 | 4.141 | 11.28 |
| 4.0855 | 17.29 | 3.9873 | 9.01 |
| 3.8254 | 6.49 | 3.7206 | 14.04 |
| 3.7489 | 10.64 | 3.5645 | 2.27 |
| 3.6983 | 14.65 | 3.5366 | 4.85 |
| 3.5817 | 3.04 | 3.3828 | 3.47 |
| 3.5064 | 9.23 | 3.2516 | 1.25 |
| 3.3392 | 4.67 | 3.134 | 0.81 |
| 3.2806 | 1.96 | 3.0848 | 0.45 |
| 3.2138 | 2.52 | 3.0638 | 1.34 |
| 3.1118 | 4.81 | 3.0111 | 3.51 |
| 3.0507 | 1.96 | 2.8739 | 0.79 |
| 2.948 | 2.40 | 2.8102 | 1.47 |
| 2.8172 | 2.89 | 2.7217 | 0.20 |
| 2.7589 | 2.27 | 2.6432 | 1.26 |
| 2.6597 | 1.86 | 2.6007 | 0.77 |
| 2.6336 | 1.10 | | |
| 2.5956 | 1.73 | | |

**TABLE - 2**

| FT-IR PEAKS OF FORM III, FORM IV AND FORM V OLANZAPINE SUMMARY OF WAVENUMBERS | | |
|---|---|---|
| FORM-III | FORM-IV | FORM-V |
| cm⁻¹ | cm⁻¹ | cm⁻¹ |
| - | 604 | 604 |
| 611 | - | - |
| 656 | 661 | - |
| 671 | - | 671 |
| 746 | - | 746 |
| 765 | 758 | 758 |
| 845 | - | 847 |
| - | 904 | - |
| 935 | 931 | 928 |
| - | - | 1357 |
| 1369 | 1365 | 1369 |
| - | 1456 | - |

**TABLE-3**

| X-RAY DIFFRACTION PEAKS OF FORM III, FORM IV AND FORM V OLANZAPINE SUMMARY OF d-spacing AND I/I₁, INTENSITY RATIO | | | | | | | | |
|---|---|---|---|---|---|---|---|---|
| FORM-III | | | FORM-IV | | | FORM-V | | |
| 2-theta [deg.] | d-spacing | I/I₁ | 2-theta [deg.] | d-spacing | I/I₁ | 2-theta [deg;] | d-spacing | I/I₁ |
| 8.5649 | 10.3156 | 100 | 8.8814 | 9.9487 | 83 | 8.3400 | 10.5932 | 17 |
| 12.3325 | 7.1713 | 16 | 10.3898 | 8.5074 | 15 | 8.6477 | 10.2170 | 100 |
| 13.6091 | 6.5014 | 17 | 10.7669 | 8.2103 | 17 | 8.8800 | 9.9503 | 57 |
| 16.0535 | 5.5165 | 24 | 18.4029 | 4.8172 | 100 | 10.3673 | 8.5259 | 22 |
| 18.2617 | 4.8541 | 46 | 18.8200 | 4.7114 | 41 | 12.4540 | 7.1016 | 17 |
| 19.4600 | 4.5578 | 24 | 19.2284 | 4.6122 | 35 | 14.5737 | 6.0731 | 17 |
| 19.7400 | 4.4938 | 38 | 19.5884 | 4.5282 | 33 | 17.0243 | 5.2041 | 19 |
| 19.9200 | 4.4536 | 36 | 20.9646 | 4.2340 | 29 | 17.7763 | 4.9856 | 20 |
| 20.8409 | 4.2588 | 49 | 21.7109 | 4.0901 | 32 | 18.4102 | 4.8153 | 62 |
| 22.2635 | 3.9898 | 52 | 23.6600 | 3.7574 | 23 | 18.6600 | 4.7514 | 34 |
| 23.8442 | 3.7288 | 42 | 24.0400 | 3.6989 | 40 | 19.5800 | 4.5302 | 24 |
| 24.9738 | 3.5626 | 25 | | | | 19.8400 | 4.4714 | 51 |
| 29.4932 | 3.0262 | 18 | | | | 20.9993 | 4.2271 | 91 |
| | | | | | | 21.4949 | 4.1307 | 40 |
| | | | | | | 22.2738 | 3.9880 | 31 |
| | | | | | | 23.5400 | 3.7763 | 10 |
| | | | | | | 23.9232 | 3.7167 | 62 |
| | | | | | | 25.1975 | 3.5315 | 22 |

**TABLE-4**

| COMPLETE FT-IR PEAKS OF FORM III, FORM IV AND FORM V OLANZAPINE SUMMARY OF WAVENUMBERS | | |
|---|---|---|
| FORM-III | FORM-IV | FORM-V |
| cm⁻¹ | cm⁻¹ | cm⁻¹ |
| - | 604 | 604 |
| 611 | - | - |
| 656 | 661 | - |
| 671 | - | 671 |
| 746 | - | 746 |
| 765 | 758 | 758 |
| 845 | - | 847 |
| - | 904 | - |
| 935 | 931 | 928 |
| 966 | 970 | 966 |
| 1008 | 1005 | 1006 |
| 1348 | 1344 | 1344 |
| - | - | 1357 |
| 1369 | 1365 | 1369 |
| 1414 | 1419 | 1414 |
| - | 1456 | 1414 |
| - | 1456 | - |
| 1469 | 1469 | 1469 |
| 1560 | 1560 | 1560 |
| 1593 | 1589 | 1585 |
| 2790 | 2798 | 2792 |
| 2837 | 2842 | 2839 |
| 2933 | 2927 | 2931 |
| 3232 | 3234 | 3228 |

**TABLE-5**

| **COMPLETE X-RAY DIFFRACTION PEAKS OF FORM III, FORM IV AND FORM V OLANZAPINE SUMMARY OF d-spacing AND I/I**_{**1**} **INTENSITY RATIO** | | | | | | | | |
|---|---|---|---|---|---|---|---|---|
| **FORM-III** | | | **FORM-IV** | | | **FORM-V** | | |
| 2-theta [deg.] | d-spacing | I/I₁ | 2-theta [deg.] | d-spacing | I/I₁ | 2-theta [deg.] | d-spacing | I/I₁ |
| 8.22 | 10.7476 | 15 | 8.88 | 9.9487 | 83 | 8.34 | 10.5932 | 17 |
| 8.56 | 10.3156 | 100 | 10.39 | 8.5074 | 15 | 8.65 | 10.2170 | 100 |
| 10.25 | 8.6245 | 11 | 10.77 | 8.2103 | 17 | 8.88 | 9.9503 | 57 |
| 12.33 | 7.1713 | 16 | 12.88 | 6.8673 | 12 | 10.37 | 8.5259 | 22 |
| 13.61 | 6.5014 | 17 | 17.82 | 4.9734 | 12 | 12.45 | 7.1016 | 17 |
| 14.48 | 6.1120 | 14 | 18.40 | 4.8172 | 100 | 14.57 | 6.0731 | 17 |
| 14.94 | 5.9251 | 12 | 18.82 | 4.7114 | 41 | 17.02 | 5.2041 | 19 |
| 15.20 | 5.8243 | 12 | 19.23 | 4.6122 | 35 | 17.78 | 4.9856 | 20 |
| 16.05 | 5.5165 | 24 | 19.59 | 4.5282 | 33 | 18.41 | 4.8153 | 62 |
| 16.92 | 5.2359 | 11 | 20.96 | 4.2340 | 29 | 18.66 | 4.7514 | 34 |
| 18.26 | 4.8541 | 46 | 21.71 | 4.0901 | 32 | 19.22 | 4.6139 | 15 |
| 18.66 | 4.7514 | 10 | 23.66 | 3.7574 | 23 | 19.58 | 4.5302 | 24 |
| 19.46 | 4.5578 | 24 | 24.04 | 3.6989 | 40 | 19.84 | 4.4714 | 51 |
| 19.74 | 4.4938 | 38 | 25.39 | 3.5052 | 11 | 20.99 | 4.2271 | 91 |
| 19.92 | 4.4536 | 36 | | | | 21.49 | 4.1307 | 40 |
| 20.84 | 4.2588 | 49 | | | | 21.80 | 4.0736 | 15 |
| 21.38 | 4.1523 | 30 | | | | 22.27 | 3.9880 | 31 |
| 21.82 | 4.0699 | 15 | | | | 23.54 | 3.7763 | 10 |
| 22.26 | 3.9898 | 52 | | | | 23.92 | 3.7167 | 62 |
| 22.81 | 3.8955 | 10 | | | | 25.20 | 3.5315 | 22 |
| 23.84 | 3.7288 | 42 | | | | 26.38 | 3.3762 | 13 |
| 24.97 | 3.5626 | 25 | | | | 29.70 | 3.0060 | 11 |
| 29.49 | 3.0262 | 18 | | | | | | |

## Claims

1. A process for producing a polymorph of olanzapine according to any of claims 11 to 19 which process comprises dissolving an initial polymorph of olanzapine in aqueous acidic solution; wherein the aqueous acidic solution comprises an organic or inorganic acid; and precipitating a different polymorph of olanzapine by neutralisation; wherein neutralisation is accomplished by the addition of an aqueous or alcoholic solution of a base.

2. A process according to claim 1, wherein the acid is hydrochloric acid, sulfuric acid, acetic acid, formic acid, citric acid, fumaric acid or maleic acid, and/or wherein the solution of the base is aqueous sodium hydroxide, alcoholic sodium hydroxide, aqueous potassium hydroxide, alcoholic potassium hydroxide or aqueous ammonia.

3. A process according to claim 1 or 2, wherein the alcoholic solution of the base comprises an alcohol selected from a mono, di, or polyhydric alcohol, preferably methanol.

4. A process according to any of claims 1 to 3, further comprising the step of recovering said different polymorph of olanzapine containing less than 5% of the initial form of olanzapine and less than 1% of other impurities.

5. A process according to any of claims 1 to 4, wherein the initial polymorph of olanzapine is Form I or Form II olanzapine, wherein Form I olanzapine is an olanzapine polymorph having a typical x-ray powder diffraction pattern represented by the following interplanar spacings:
| d-spacings (Å) |
|---|
| 9.9463 |
| 8.5579 |
| 8.2445 |
| 6.8862 |
| 6.3787 |
| 6.2439 |
| 5.5895 |
| 5.3055 |
| 4.9815 |
| 4.8333 |
| 4.7255 |
| 4.6286 |
| 4.533 |
| 4.4624 |
| 4.2915 |
| 4.2346 |
| 4.0855 |
| 3.8254 |
| 3.7489 |
| 3.6983 |
| 3.5817 |
| 3.5064 |
| 3.3392 |
| 3.2806 |
| 3.2138 |
| 3.1118 |
| 3.0507 |
| 2.948 |
| 2.8172 |
| 2.7589 |
| 2.6597 |
| 2.6336 |
| 2.5956; |
and Form II olanzapine is an olanzapine polymorph having a typical x-ray powder diffraction pattern represented by the following interplanar spacings:
| d-spacings (Å) |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007. |

6. A process according to any of claims 1 to 5, wherein the different polymorph of olanzapine is Form III olanzapine having a typical x-ray powder diffraction pattern as represented by the following interplanar spacings:
| d-spacings (Å) |
|---|
| 10.3156 |
| 7.1713 |
| 6.5014 |
| 5.5165 |
| 4.8541 |
| 4.5578 |
| 4.4938 |
| 4.4536 |
| 4.2588 |
| 3.9898 |
| 3.7288 |
| 3.5626 |
| 3.0262 |
or wherein the different polymorph of olanzapine is Form IV olanzapine having a typical x-ray powder diffraction pattern as represented by the following interplanar spacings:
| d-spacings (Å) |
|---|
| 9.9487 |
| 8.5074 |
| 8.2103 |
| 4.8172 |
| 4.7114 |
| 4.6122 |
| 4.5282 |
| 4.2340 |
| 4.0901 |
| 3.7574 |
| 3.6989 |
or wherein the different polymorph of olanzapine is Form V olanzapine having a typical x-ray powder diffraction pattern as represented by the following interplanar spacings:
| d-spacings (Å) |
|---|
| 10.5932 |
| 10.2170 |
| 9.9503 |
| 8.5259 |
| 7.1016 |
| 6.0731 |
| 5.2041 |
| 4.9856 |
| 4.8153 |
| 4.7514 |
| 4.5302 |
| 4.4714 |
| 4.2271 |
| 4.1307 |
| 3.9880 |
| 3.7763 |
| 3.7167 |
| 3.5315 |

7. A process according to any preceding claim, wherein the precipitation is conducted at a temperature between about 0°C and about 100°C, or between about 0°C and about 35°C, or between about 10°C and about 30°C.

8. A process according to any preceding claim, wherein the precipitation comprises adjusting the pH to between about 6 and about 12, or between about 8 and about 11.

9. A process according to any preceding claim, wherein the acidic solution comprises from about 5% to about 50% acid, optionally wherein the acidic solution is about 50% acetic acid and the basic solution is about 15% aqueous ammonia.

10. A process according to any preceding claim, wherein the acidic solution is about 38% formic acid and the basic solution is about 10% methanolic sodium hydroxide, or wherein the acidic solution is about 10% hydrochloric acid and the basic solution is about 10% aqueous sodium hydroxide, or wherein the acidic solution is about 43% acetic acid and the basic solution is about 25% aqueous ammonia, or wherein the acidic solution is about 40% acetic acid and the basic solution is about 50% aqueous sodium hydroxide, or wherein the acidic solution is about 20% formic acid and the basic solution is about 25% aqueous ammonia, or wherein the acidic solution is about 33% acetic acid and the basic solution is about 50% aqueous ammonia, or wherein the acidic solution is about 50% acetic acid and the basic solution is about 25% aqueous ammonia.

11. Form III olanzapine polymorph having a typical x-ray powder diffraction pattern as represented by the following interplanar spacings:
| d-spacings (Å) |
|---|
| 10.3156 |
| 7.1713 |
| 6.5014 |
| 5.5165 |
| 4.8541 |
| 4.5578 |
| 4.4938 |
| 4.4536 |
| 4.2588 |
| 4.0699 |
| 3.9898 |
| 3.7288 |
| 3.5626 |
| 3.0262 |
optionally further **characterized by** substantially the following x-ray powder diffraction pattern, wherein d represents the interplanar spacing and I/I₁ represents the typical relative intensities:
| d-spacing (Å) | I/I₁ |
|---|---|
| 10.3156 | 100 |
| 7.1713 | 16 |
| 6.5014 | 17 |
| 5.5165 | 24 |
| 4.8541 | 46 |
| 4.5578 | 24 |
| 4.4938 | 38 |
| 4.4536 | 36 |
| 4.2588 | 49 |
| 3.9898 | 52 |
| 3.7288 | 42 |
| 3.5626 | 25 |
| 3.0262 | 18. |

12. Form III olanzapine polymorph according to claim 11, further **characterized by** having an infrared spectrum having absorbances at the following wavenumbers:
| |
|---|
| 611 |
| 656 |
| 671 |
| 746 |
| 765 |
| 845 |
| 935 |
| 1369. |

13. Form III olanzapine polymorph according to claim 11 or 12 produced by the process of either dissolving Form I or Form II olanzapine in 50% aqueous acetic acid, and precipitating substantially pure Form III olanzapine with 15% aqueous ammonia; or by dissolving Form I or Form II olanzapine in about 33% aqueous acetic acid, and precipitating substantially pure Form III olanzapine with about 50% aqueous ammonia; wherein Form I olanzapine is an olanzapine polymorph having a typical x-ray powder diffraction pattern represented by the following interplanar spacings:
| d-spacings (Å) |
|---|
| 9.9463 |
| 8.5579 |
| 8.2445 |
| 6.8862 |
| 6.3787 |
| 6.2439 |
| 5.5895 |
| 5.3055 |
| 4.9815 |
| 4.8333 |
| 4.7255 |
| 4.6286 |
| 4.533 |
| 4.4624 |
| 4.2915 |
| 4.2346 |
| 4.0855 |
| 3.8254 |
| 3.7489 |
| 3.6983 |
| 3.5817 |
| 3.5064 |
| 3.3392 |
| 3.2806 |
| 3.2138 |
| 3.1118 |
| 3.0507 |
| 2.948 |
| 2.8172 |
| 2.7589 |
| 2.6597 |
| 2.6336 |
| 2.5956; |
and Form II olanzapine is an olanzapine polymorph having a typical x-ray powder diffraction pattern represented by the following interplanar spacings:
| d-spacings (Å) |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007. |

14. Form IV olanzapine polymorph having a typical x-ray powder diffraction pattern as represented by the following interplanar spacings:
| d-spacings (Å) |
|---|
| 9.9487 |
| 8.5074 |
| 8.2103 |
| 4.8172 |
| 4.7114 |
| 4.6122 |
| 4.5282 |
| 4.2340 |
| 4.0901 |
| 3.7574 |
| 3.6989. |
optionally further **characterized by** substantially the following x-ray powder diffraction pattern, wherein d represents the interplanar spacing and I/I₁ represents the typical relative intensities:
| d-spacing (Å) | I/I₁ |
|---|---|
| 9.9487 | 83 |
| 8.5074 | 15 |
| 8.2103 | 17 |
| 4.8172 | 100 |
| 4.7114 | 41 |
| 4.6122 | 35 |
| 4.5282 | 33 |
| 4.2340 | 29 |
| 4.0901 | 32 |
| 3.7574 | 23 |
| 3.6989 | 40 |

15. Form IV olanzapine polymorph according to claim 14 further **characterized by** having an infrared spectrum having absorbances at the following wavenumbers:
| |
|---|
| 604 |
| 661 |
| 758 |
| 904 |
| 931 |
| 1365 |
| 1456. |

16. Form IV olanzapine polymorph according to claim 14 or 15 produced by the process of either dissolving Form I or Form II olanzapine in about 38% aqueous formic acid, and precipitating substantially pure Form IV olanzapine using about 10% methanolic sodium hydroxide; or by dissolving Form I or Form II olanzapine in about 43% aqueous acetic acid, and precipitating substantially pure Form IV olanzapine using about 25% aqueous ammonia; wherein Form I olanzapine is an olanzapine polymorph having a typical x-ray powder diffraction pattern represented by the following interplanar spacings:
| d-spacings (Å) |
|---|
| 9.9463 |
| 8.5579 |
| 8.2445 |
| 6.8862 |
| 6.3787 |
| 6.2439 |
| 5.5895 |
| 5.3055 |
| 4.9815 |
| 4.8333 |
| 4.7255 |
| 4.6286 |
| 4.533 |
| 4.4624 |
| 4.2915 |
| 4.2346 |
| 4.0855 |
| 3.8254 |
| 3.7489 |
| 3.6983 |
| 3.5817 |
| 3.5064 |
| 3.3392 |
| 3.2806 |
| 3.2138 |
| 3.1118 |
| 3.0507 |
| 2.948 |
| 2.8172 |
| 2.7589 |
| 2.6597 |
| 2.6336 |
| 2.5956; |
and Form II olanzapine is an olanzapine polymorph having a typical x-ray powder diffraction pattern represented by the following interplanar spacings:
| d-spacings (Å) |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007. |

17. Form V olanzapine polymorph having a typical x-ray powder diffraction pattern as represented by the following interplanar spacings:
| d-spacings (Å) |
|---|
| 10.5932 |
| 10.2170 |
| 9.9503 |
| 8.5259 |
| 7.1016 |
| 6.0731 |
| 5.2041 |
| 4.9856 |
| 4.8153 |
| 4.7514 |
| 4.5302 |
| 4.4714 |
| 4.2271 |
| 4.1307 |
| 3.9880 |
| 3.7763 |
| 3.7167 |
| 3.5315. |
optionally further **characterized by** substantially the following x-ray powder diffraction pattern, wherein d represents the interplanar spacing and I/I₁, represents the typical relative intensities:
| d-spacing (Å) | I/I₁ |
|---|---|
| 10.5932 | 17 |
| 10.2170 | 100 |
| 9.9503 | 57 |
| 8.5259 | 22 |
| 7.1016 | 17 |
| 6.0731 | 17 |
| 5.2041 | 19 |
| 4.9856 | 20 |
| 4.8153 | 62 |
| 4.7514 | 34 |
| 4.5302 | 24 |
| 4.4714 | 51 |
| 4.2271 | 91 |
| 4.1307 | 40 |
| 3.9880 | 31 |
| 3.7763 | 10 |
| 3.7167 | 62 |
| 3.5315 | 22 |

18. Form V olanzapine polymorph according to claim 17, further **characterized by** having an infrared spectrum having absorbances at the following wavenumbers:
| |
|---|
| 604 |
| 671 |
| 746 |
| 758 |
| 847 |
| 928 |
| 1357 |
| 1369. |

19. Form V olanzapine polymorph according to claim 17 or 18 produced by the process of either dissolving Form I or Form II olanzapine in about 10% aqueous hydrochloric acid, and precipitating substantially pure Form V olanzapine using about 10% aqueous sodium hydroxide; or by dissolving Form I or Form II olanzapine in about 40% acetic acid, and precipitating substantially pure Form V olanzapine using about 50% aqueous sodium hydroxide; or by dissolving Form I or Form II olanzapine in about 20% aqueous formic acid, and precipitating substantially pure Form V olanzapine using about 25% aqueous ammonia; or by dissolving Form I or Form II olanzapine in about 50% aqueous acetic acid, and precipitating substantially pure Form V olanzapine using about 25% aqueous ammonia; wherein Form I olanzapine is an olanzapine polymorph having a typical x-ray powder diffraction pattern represented by the following interplanar spacings:
| d-spacings (Å) |
|---|
| 9.9463 |
| 8.5579 |
| 8.2445 |
| 6.8862 |
| 6.3787 |
| 6.2439 |
| 5.5895 |
| 5.3055 |
| 4.9815 |
| 4.8333 |
| 4.7255 |
| 4.6286 |
| 4.533 |
| 4.4624 |
| 4.2915 |
| 4.2346 |
| 4.0855 |
| 3.8254 |
| 3.7489 |
| 3.6983 |
| 3.5817 |
| 3.5064 |
| 3.3392 |
| 3.2806 |
| 3.2138 |
| 3.1118 |
| 3.0507 |
| 2.948 |
| 2.8172 |
| 2.7589 |
| 2.6597 |
| 2.6336 |
| 2.5956; |
and Form II olanzapine is an olanzapine polymorph having a typical x-ray powder diffraction pattern represented by the following interplanar spacings:
| d-spacings (Å) |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007. |

20. A pharmaceutical formulation containing as an active ingredient at least one olanzapine polymorph associated with one or more pharmaceutically acceptable carriers, excipients or diluents therefor; wherein the at least one olanzapine polymorph is Form III olanzapine, Form IV olanzapine or Form V olanzapine, and salts and mixtures thereof; and wherein Forms III, IV and V olanzapine are olanzapine polymorphs having typical x-ray powder diffraction patterns represented by the following interplanar spacings:
| **FORM-III** | **FORM-IV** | **FORM IV** |
|---|---|---|
| d-spacing (Å) | d-spacing (Å) | d-spacing (A) |
| 10.3156 | 9.9487 | 10.5932 |
| 7.1713 | 8.5074 | 10.2170 |
| 6.5014 | 8.2103 | 9.9503 |
| 5.5165 | 4.8172 | 8.5259 |
| 4.8541 | 4.7114 | 7.1016 |
| 4.5578 | 4.6122 | 6.0731 |
| 4.4938 | 4.5282 | 5.2041 |
| 4.4536 | 4.2340 | 4.9856 |
| 4.2588 | 4.0901 | 4.8153 |
| 3.9898 | 3.7574 | 4.7514 |
| 3.7288 | 3.6989 | 4.5302 |
| 3.5626 | | 4.4714 |
| 3.0262 | | 4.2271 |
| | | 4.1307 |
| | | 3.9880 |
| | | 3.7763 |
| | | 3.7167 |
| | | 3.5315 |

21. Use of Form III, Form IV or Form V olanzapine as claimed in any of claims 11 to 20, and salts and mixtures thereof, for the manufacture of a medicament for treating a psychotic condition, mild anxiety or gastrointestinal conditions.

## Patentansprüche

1. Verfahren zum Herstellen einer polymorphen Form von Olanzapin gemäß einem der Ansprüche 11 bis 19, wobei das Verfahren umfasst das Auflösen einer anfänglichen polymorphen Form von Olanzapin in wässriger saurer Lösung, worin die wässrige saure Lösung eine organische oder anorganische Säure umfasst, und das Ausfällen einer anderen polymorphen Form von Olanzapin durch Neutralisation, worin die Neutralisation durch die Zugabe einer wässrigen oder alkoholischen Lösung einer Base erfolgt.

2. Verfahren gemäß Anspruch 1, worin die Säure Chlorwasserstoffsäure, Schwefelsäure, Essigsäure, Ameisensäure, Citronensäure, Fumarsäure oder Maleinsäure ist, und/oder worin die Lösung der Base wässriges Natriumhydroxid, alkoholisches Natriumhydroxid, wässriges Kaliumhydroxid, alkoholisches Kaliumhydroxid oder wässriges Ammoniak ist.

3. Verfahren gemäß Anspruch 1 oder 2, worin die alkoholische Lösung der Base einen Alkohol umfasst, ausgewählt aus einem ein-, zwei- oder mehrwertigen Alkohol, bevorzugt Methanol.

4. Verfahren gemäß einem der Ansprüche 1 bis 3, das weiter den Schritt des Rückgewinnens der anderen polymorphen Form von Olanzapin umfasst, die weniger als 5 % der anfänglichen Form von Olanzapin und weniger als 1 % anderer Verunreinigungen enthält.

5. Verfahren gemäß einem der Ansprüche 1 bis 4, worin die anfängliche polymorphe Form von Olanzapin Olanzapin der Form I oder der Form II ist, worin Olanzapin der Form I eine polymorphe Olanzapinform mit einem typischen Pulverröntgenbeugungsbild ist, wiedergegeben durch die folgenden Gitterebenenabstände:
| d-Abstände (Å) |
|---|
| 9,9463 |
| 8,5579 |
| 8,2445 |
| 6,8862 |
| 6,3787 |
| 6,2439 |
| 5,5895 |
| 5,3055 |
| 4,9815 |
| 4,8333 |
| 4,7255 |
| 4,6286 |
| 4,533 |
| 4,4624 |
| 4,2915 |
| 4,2346 |
| 4,0855 |
| 3,8254 |
| 3,7489 |
| 3,6983 |
| 3,5817 |
| 3,5064 |
| 3,3392 |
| 3,2806 |
| 3,2138 |
| 3,1118 |
| 3,0507 |
| 2,948 |
| 2,8172 |
| 2,7589 |
| 2,6597 |
| 2,6336 |
| 2,5956, |
und Olanzapin der Form II eine polymorphe Olanzapinform mit einem typischen Pulverröntgenbeugungsbild ist, wiedergegeben durch die folgenden Gitterebenenabstände:
| d-Abstände (Å) |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516 |
| 3,134 |
| 3,0848 |
| 3,0638 |
| 3,0111 |
| 2,8739 |
| 2,8102 |
| 2,7217 |
| 2,6432 |
| 2,6007, |

6. Verfahren gemäß einem der Ansprüche 1 bis 5, worin die andere polymorphe Form von Olanzapin Olanzapin der Form III mit einem typischen Pulverröntgenbeugungsbild ist, wie durch die folgenden Gitterebenenabstände wiedergegeben:
| d-Abstände (Å) |
|---|
| 10,3156 |
| 7,1713 |
| 6,5014 |
| 5,5165 |
| 4,8541 |
| 4,5578 |
| 4,4938 |
| 4,4536 |
| 4,2588 |
| 3,9898 |
| 3,7288 |
| 3,5626 |
| 3,0262, |
oder worin die andere polymorphe Form von Olanzapin Olanzapin der Form IV mit einem typischen Pulverröntgenbeugungsbild ist, wie durch die folgenden Gitterebenenabstände wiedergegeben:
| d-Abstände (Å) |
|---|
| 9,9487 |
| 8,5074 |
| 8,2103 |
| 4,8172 |
| 4,7114 |
| 4,6122 |
| 4,5282 |
| 4,2340 |
| 4,0901 |
| 3,7574 |
| 3,6989, |
oder worin die andere polymorphe Form von Olanzapin Olanzapin der Form V mit einem typischen Pulverröntgenbeugungsbild ist, wie durch die folgenden Gitterebenenabstände wiedergegeben:
| d-Abstände (Å) |
|---|
| 10,5932 |
| 10,2170 |
| 9,9503 |
| 8,5259 |
| 7,1016 |
| 6,0731 |
| 5,2041 |
| 4,9856 |
| 4,8153 |
| 4,7514 |
| 4,5302 |
| 4,4714 |
| 4,2271 |
| 4,1307 |
| 3,9880 |
| 3,7763 |
| 3,7167 |
| 3,5315 |

7. Verfahren gemäß einem der vorhergehenden Ansprüche, worin die Ausfällung bei einer Temperatur zwischen etwa 0°C und etwa 100°C oder zwischen etwa 0°C und etwa 35°C oder zwischen etwa 10°C und etwa 30°C durchgeführt wird.

8. Verfahren gemäß einem der vorhergehenden Ansprüche, worin die Ausfällung das Einstellen des pH auf zwischen etwa 6 und etwa 12 oder zwischen etwa 8 und etwa 11 umfasst.

9. Verfahren gemäß einem der vorhergehenden Ansprüche, worin die saure Lösung etwa 5 % bis etwa 50 % Säure umfasst, optional worin die saure Lösung etwa 50 %ige Essigsäure ist, und die basische Lösung etwa 15 %iges wässriges Ammoniak ist.

10. Verfahren gemäß einem der vorhergehenden Ansprüche, worin die saure Lösung etwa 38 %ige Ameisensäure ist, und die basische Lösung etwa 10 %iges methanolisches Natriumhydroxid ist, oder worin die saure Lösung etwa 10 %ige Chlorwasserstoffsäure ist, und die basische Lösung etwa 10 %iges wässriges Natriumhydroxid ist, oder worin die saure Lösung etwa 43 %ige Essigsäure ist, und die basische Lösung etwa 25 %iges wässriges Ammoniak ist, oder worin die saure Lösung etwa 40 %ige Essigsäure ist, und die basische Lösung etwa 50 %iges wässriges Natriumhydroxid ist, oder worin die saure Lösung etwa 20 %ige Ameisensäure ist, und die basische Lösung etwa 25 %iges wässriges Ammoniak ist, oder worin die saure Lösung etwa 33 %ige Essigsäure ist, und die basische Lösung etwa 50 %iges wässriges Ammoniak ist, oder worin die saure Lösung etwa 50 %ige Essigsäure ist, und die basische Lösung etwa 25 %iges wässriges Ammoniak ist.

11. Polymorphes Olanzapin der Form III mit einem typischen Pulverröntgenbeugungsbild, wie durch die folgenden Gitterebenenabstände wiedergegeben:
| d-Abstände (Å) |
|---|
| 10,3156 |
| 7,1713 |
| 6,5014 |
| 5,5165 |
| 4,8541 |
| 4,5578 |
| 4,4938 |
| 4,4536 |
| 4,2588 |
| 4,0699 |
| 3,9898 |
| 3,7288 |
| 3,5626 |
| 3,0262, |
optional weiter **gekennzeichnet durch** im Wesentlichen das folgende Pulverröntgenbeugungsbild, worin d den Gitterebenenabstand und I/I₁ die typischen relativen Intensitäten wiedergibt:
| d-Abstände (Å) | I/I₁ |
|---|---|
| 10,3156 | 100 |
| 7,1713 | 16 |
| 6,5014 | 17 |
| 5,5165 | 24 |
| 4,8541 | 46 |
| 4,5578 | 24 |
| 4,4938 | 38 |
| 4,4536 | 36 |
| 4,2588 | 49 |
| 3,9898 | 52 |
| 3,7288 | 42 |
| 3,5626 | 25 |
| 3,0262 | 18. |

12. Polymorphes Olanzapin der Form III gemäß Anspruch 11, weiter **gekennzeichnet durch** ein Infrarotspektrum mit Absorptionen bei den folgenden Wellenzahlen:
| |
|---|
| 611 |
| 656 |
| 671 |
| 746 |
| 765 |
| 845 |
| 935 |
| 1369. |

13. Polymorphes Olanzapin der Form III gemäß Anspruch 11 oder 12, hergestellt durch das Verfahren von entweder Auflösen von Olanzapin der Form I oder der Form II in 50 %iger wässriger Essigsäure und Ausfällen von im Wesentlichen reinem Olanzapin der Form III mit 15 %igem wässrigem Ammoniak, oder durch Auflösen von Olanzapin der Form I oder der Form II in etwa 33 %iger wässriger Essigsäure und Ausfällen von im Wesentlichen reinem Olanzapin der Form III mit etwa 50 %igem wässrigem Ammoniak, worin Olanzapin der Form I ein polymorphes Olanzapin mit einem typischen Pulverröntgenbeugungsbild ist, wiedergegeben durch die folgenden Gitterebenenabstände:
| d-Abstände (Å) |
|---|
| 9,9463 |
| 8,5579 |
| 8,2445 |
| 6,8862 |
| 6,3787 |
| 6,2439 |
| 5,5895 |
| 5,3055 |
| 4,9815 |
| 4,8333 |
| 4,7255 |
| 4,6286 |
| 4,533 |
| 4,4624 |
| 4,2915 |
| 4,2346 |
| 4,0855 |
| 3,8254 |
| 3,7489 |
| 3, 6983 |
| 3,5817 |
| 3,5064 |
| 3,3392 |
| 3,2806 |
| 3,2138 |
| 3,1118 |
| 3,0507 |
| 2,948 |
| 2,8172 |
| 2, 7589 |
| 2,6597 |
| 2,6336 |
| 2,5956, |
und Olanzapin der Form II ein polymorphes Olanzapin mit einem typischen Pulverröntgenbeugungsbild ist, wiedergegeben durch die folgenden Gitterebenenabstände:
| d-Abstände (Å) |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516 |
| 3,134 |
| 3,0848 |
| 3,0638 |
| 3,0111 |
| 2,8739 |
| 2,8102 |
| 2,7217 |
| 2,6432 |
| 2,6007. |

14. Polymorphes Olanzapin der Form IV mit einem typischen Pulverröntgenbeugungsbild, wie durch die folgenden Gitterebenenabstände wiedergegeben:
| d-Abstände (Å) |
|---|
| 9,9487 |
| 8,5074 |
| 8,2103 |
| 4,8172 |
| 4,7114 |
| 4,6122 |
| 4,5282 |
| 4,2340 |
| 4,0901 |
| 3,7574 |
| 3,6989, |
optional weiter **gekennzeichnet durch** im Wesentlichen das folgende Pulverröntgenbeugungsbild, worin d den Gitterebenenabstand bedeutet, und I/I₁ die typischen relativen Intensitäten bedeutet:
| d-Abstände (Å) | I/I₁ |
|---|---|
| 9,9487 | 83 |
| 8,5074 | 15 |
| 8,2103 | 17 |
| 4,8172 | 100 |
| 4,7114 | 41 |
| 4,6122 | 35 |
| 4,5282 | 33 |
| 4,2340 | 29 |
| 4,0901 | 32 |
| 3,7574 | 23 |
| 3,6989 | 40 |

15. Polymorphes Olanzapin der Form IV gemäß Anspruch 14, weiter **gekennzeichnet durch** ein Infrarotspektrum mit Absorptionen bei den folgenden Wellenzahlen:
| |
|---|
| 604 |
| 661 |
| 758 |
| 904 |
| 931 |
| 1365 |
| 1456. |

16. Polymorphes Olanzapin der Form IV gemäß Anspruch 14 oder 15, hergestellt durch das Verfahren von entweder Auflösen von Olanzapin der Form I oder der Form II in etwa 38 %iger Ameisensäure und Ausfällen von im Wesentlichen reinem Olanzapin der Form IV unter Verwendung von etwa 10 %igem methanolischem Natriumhydroxid oder durch Auflösen von Olanzapin der Form I oder der Form II in etwa 43 %iger wässriger Essigsäure und Ausfällen von im Wesentlichen reinem Olanzapin der Form IV unter Verwendung von etwa 25 %igem wässrigem Ammoniak, worin Olanzapin der Form I ein polymorphes Olanzapin mit einem typischen Pulverröntgenbeugungsbild ist, wiedergegeben durch die folgenden Gitterebenenabstände:
| d-Abstände (Å) |
|---|
| 9,9463 |
| 8,5579 |
| 8,2445 |
| 6,8862 |
| 6,3787 |
| 6, 2439 |
| 5,5895 |
| 5,3055 |
| 4,9815 |
| 4,8333 |
| 4,7255 |
| 4,6286 |
| 4,533 |
| 4,4624 |
| 4,2915 |
| 4,2346 |
| 4,0855 |
| 3,8254 |
| 3, 7489 |
| 3,6983 |
| 3,5817 |
| 3,5064 |
| 3,3392 |
| 3,2806 |
| 3,2138 |
| 3,1118 |
| 3,0507 |
| 2,948 |
| 2.8172 |
| 2.7589 |
| 2.6597 |
| 2,6336 |
| 2,5956, |
und Olanzapin der Form II ein polymorphes Olanzapin mit einem typischen Pulverröntgenbeugungsbild ist, wiedergegeben durch die folgenden Gitterebenenabstände:
| d-Abstände (Å) |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516 |
| 3,134 |
| 3,0848 |
| 3,0638 |
| 3,0111 |
| 2,8739 |
| 2,8102 |
| 2,7217 |
| 2,6432 |
| 2,6007. |

17. Polymorphes Olanzapin der Form V mit einem typischen Pulverröntgenbeugungsbild, wie durch die folgenden Gitterebenenabstände wiedergegeben:
| d-Abstände (Å) |
|---|
| 10,5932 |
| 10,2170 |
| 9,9503 |
| 8,5259 |
| 7,1016 |
| 6.0731 |
| 5,2041 |
| 4,9856 |
| 4,8153 |
| 4,7514 |
| 4,5302 |
| 4,4714 |
| 4,2271 |
| 4,1307 |
| 3,9880 |
| 3,7763 |
| 3,7167 |
| 3,5315, |
optional weiter **gekennzeichnet durch** im Wesentlichen das folgende Pulverröntgenbeugungsbild, worin d den Gitterebenenabstand bedeutet, und I/I₁ die typischen relativen Intensitäten bedeutet:
| d-Abstände (Å) | I/I₁ |
|---|---|
| 10,5932 | 17 |
| 10,2170 | 100 |
| 9,9503 | 57 |
| 8,5259 | 22 |
| 7,1016 | 17 |
| 6,0731 | 17 |
| 5,2041 | 19 |
| 4,9856 | 20 |
| 4,8153 | 62 |
| 4,7514 | 34 |
| 4,5302 | 24 |
| 4,4714 | 51 |
| 4,2271 | 91 |
| 4,1307 | 40 |
| 3,9880 | 31 |
| 3,7763 | 10 |
| 3,7167 | 62 |
| 3,5315 | 22. |

18. Polymorphes Olanzapin der Form V gemäß Anspruch 17, weiter **gekennzeichnet durch** ein Infrarotspektrum mit Absorptionen bei den folgenden Wellenzahlen:
| |
|---|
| 604 |
| 671 |
| 746 |
| 758 |
| 847 |
| 928 |
| 1357 |
| 1369. |

19. Polymorphes Olanzapin der Form V gemäß Anspruch 17 oder 18, hergestellt durch das Verfahren von entweder Auflösen von Olanzapin der Form I oder der Form II in etwa 10 %iger wässriger Chlorwasserstoffsäure und Ausfällen von im Wesentlichen reinem Olanzapin der Form V unter Verwendung von etwa 10 %igem wässrigem Natriumhydroxid oder durch Auflösen von Olanzapin der Form I oder der Form II in etwa 40 %iger wässriger Essigsäure und Ausfällen von im Wesentlichen reinem Olanzapin der Form V unter Verwendung von 50 %igem wässrigem Natriumhydroxid oder durch Auflösen von Olanzapin der Form I oder der Form II in etwa 20 %iger wässriger Ameisensäure und Ausfällen von im Wesentlichen reinem Olanzapin der Form V unter Verwendung von etwa 25 %igem wässrigem Ammoniak oder durch Auflösen von Olanzapin der Form I oder der Form II in etwa 50 %iger wässriger Essigsäure und Ausfällen von im Wesentlichen reinem Olanzapin der Form V unter Verwendung von etwa 25 %igem wässrigem Ammoniak, worin Olanzapin der Form I ein polymorphes Olanzapin mit einem typischen Pulverröntgenbeugungsbild ist, wiedergegeben durch die folgenden Gitterebenenabstände:
| d-Abstände (Å) |
|---|
| 9,9463 |
| 8,5579 |
| 8,2445 |
| 6,8862 |
| 6,3787 |
| 6,2439 |
| 5,5895 |
| 5,3055 |
| 4,9815 |
| 4,8333 |
| 4,7255 |
| 4,6286 |
| 4,533 |
| 4,4624 |
| 4,2915 |
| 4,2346 |
| 4,0855 |
| 3,8254 |
| 3,7489 |
| 3,6983 |
| 3,5817 |
| 3,5064 |
| 3,3392 |
| 3,2806 |
| 3,2138 |
| 3,1118 |
| 3,0507 |
| 2,948 |
| 2,8172 |
| 2,7589 |
| 2,6597 |
| 2,6336 |
| 2,5956, |
und Olanzapin der Form II ein polymorphes Olanzapin mit einem typischen Pulverröntgenbeugungsbild ist, wiedergegeben durch die folgenden Gitterebenenabstände:
| d-Abstände (Å) |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516 |
| 3,134 |
| 3,0848 |
| 3,0638 |
| 3,0111 |
| 2,8739 |
| 2,8102 |
| 2,7217 |
| 2,6432 |
| 2,6007. |

20. Pharmazeutische Formulierung, enthaltend als aktiven Bestandteil wenigstens ein polymorphes Olanzapin, vereinigt mit einem oder mehreren pharmazeutisch annehmbaren Trägern, Arzneimittelträgem oder Verdünnungsmitteln dafür, worin das wenigstens eine polymorphe Olanzapin Olanzapin der Form III, Olanzapin der Form IV oder Olanzapin der Form V ist, und Salze und Mischungen davon, und worin Olanzapin der Formen III, IV und V polymorphe Formen von Olanzapin mit typischen Pulverröntgenbeugungsbildern sind, wiedergegeben durch die folgenden Gitterebenenabstände:
| **FORM III** | **FORM IV** | **FORM V** |
|---|---|---|
| d-Abstand (Å) | d-Abstand (Å) | d-Abstand (Å) |
| 10,3156 | 9,9487 | 10,5932 |
| 7,1713 | 8,5074 | 10,2170 |
| 6,5014 | 8,2103 | 9,9503 |
| 5,5165 | 4,8172 | 8,5259 |
| 4,8541 | 4,7114 | 7,1016 |
| 4,5578 | 4,6122 | 6,0731 |
| 4,4938 | 4,5282 | 5,2041 |
| 4,4536 | 4,2340 | 4,9856 |
| 4,2588 | 4,0901 | 4,8153 |
| 3,9898 | 3,7574 | 4,7514 |
| 3,7288 | 3,6989 | 4,5302 |
| 3,5626 | | 4,4714 |
| 3,0262 | | 4,2271 |
| | | 4,1307 |
| | | 3,9880 |
| | | 3,7763 |
| | | 3,7167 |
| | | 3,5315 |

21. Verwendung von Olanzapin der Form III, der Form IV oder der Form V, wie in einem der Ansprüche 11 bis 20 beansprucht, und Salze und Mischungen davon, zur Herstellung eines Medikaments zur Behandlung eines psychotischen Zustands, eines schwachen Angstzustands oder gastrointestinaler Zustände.

## Revendications

1. Procédé de production d'une forme polymorphe d'olanzapine selon l'une quelconque des revendications 11 à 19, lequel procédé comprend la dissolution d'une forme polymorphe d'olanzapine initiale dans une solution acide aqueuse, la solution acide aqueuse comprenant un acide organique ou inorganique, et la précipitation d'une forme polymorphe d'olanxapine différente par neutralisation, la neutralisation se faisant par l'ajout d'une solution aqueuse ou alcoolique d'une base.

2. Procédé selon la revendication 1, dans lequel l'acide est l'acide chlorhydrique, l'acide sulfurique, l'acide acétique, l'acide formique, l'acide citrique, l'acide fiunarique ou l'acide maléique, et/ou dans lequel la solution de la base est de l'hydroxyde de sodium aqueux, de l'hydroxyde de sodium alcoolique, de l'hydroxyde de potassium aqueux, de l'hydroxyde de potassium alcoolique ou de l'ammoniaque aqueux.

3. Procédé selon la revendication 1 ou la revendication 2, dans lequel la solution alcoolique de la base comprend un alcool choisi parmi un mono-, di- ou poly-alcool, de préférence le méthanol.

4. Procédé selon l'une quelconque des revendi cations 1 à 3, comprenant en plus l'étape de récupération de ladite forme polymorphe d'olanzapine différente contenant moins de 5% de la forme d'olanzapine initiale et moins de 1% d'autres impuretés.

5. Procédé selon l'une quelconque des revendications 1 à 4, dans lequel la forme polymorphe d'olanzapine initiale est de l'olanzapine de forme I ou de forme II, l'olanzapine de forme I étant une forme polymorphe d'olanzapine ayant un spectre typique de diffraction des rayons X par poudre représenté par les écartements de plans suivants :
| Ecartements d (Å) |
|---|
| 9,9463 |
| 8,5579 |
| 8,2445 |
| 6,8862 |
| 6,3787 |
| 6,2439 |
| 5,5895 |
| 5,3055 |
| 4,9815 |
| 4,8333 |
| 4,7255 |
| 4,6286 |
| 4,533 |
| 4,4624 |
| 4,2915 |
| 4,2346 |
| 4,0855 |
| 3,8254 |
| 3,7489 |
| 3,6983 |
| 3,5817 |
| 3,5064 |
| 3,3392 |
| 3,2806 |
| 3,2138 |
| 3,1118 |
| 3,0507 |
| 2,948 |
| 2,8172 |
| 2,7589 |
| 2,6597 |
| 2,6336 |
| 2,5956; |
et l'olanzapinc de forme II étant une forme polymorphe d'olanzapine ayant un spectre typique de diffraction des rayons X par poudre représenté par les écartements de plans suivants :
| Ecartements d (Å) |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516 |
| 3,134 |
| 3,0848 |
| 3,0638 |
| 3,0111 |
| 2,8739 |
| 2,8102 |
| 2,7217 |
| 2,6432 |
| 2,6007. |

6. Procédé selon l'une quelconque des revendications 1 à 5, dans lequel la forme polymorphe d'olanzapine différente est de l'olanzapine de forme III ayant un spectre typique de diffraction des rayons X par poudre représenté par les écartements de plans suivants :
| Ecartements d (Å) |
|---|
| 10,3156 |
| 7,1713 |
| 6,5014 |
| 5,5165 |
| 4,8541 |
| 4,5578 |
| 4,4938 |
| 4,4536 |
| 4,2588 |
| 3,9898 |
| 3,7288 |
| 3,5626 |
| 3,0262 |
ou dans lequel la forme polymorphe d'olanzapine différente est de l'olanzapine de forme IV ayant un spectre typique de diffraction des rayons X par poudre représenté par les écartements de plans suivants :
| Ecartements d (Å) |
|---|
| 9,9487 |
| 8,5074 |
| 8,2103 |
| 4,8172 |
| 4,7114 |
| 4,6122 |
| 4,5282 |
| 4,2340 |
| 4,0901 |
| 3,7574 |
| 3,6989 |
ou dans lequel la forme polymorphe d'olanzapine différente est de l'olanzapine de forme V ayant un spectre typique de diffraction des rayons X par poudre représenté par les écartements de plans suivants :
| Ecartements d (Å) |
|---|
| 10,5932 |
| 10,2170 |
| 9,9503 |
| 8,5259 |
| 7,1016 |
| 6,0731 |
| 5,2041 |
| 4,9856 |
| 4,8153 |
| 4,7514 |
| 4,5302 |
| 4,4714 |
| 4,2271 |
| 4,1307 |
| 3,9880 |
| 3,7763 |
| 3,7167 |
| 3,5315 |

7. Procédé selon l'une quelconque des revendications précédentes, dans lequel la précipitation s'effectue à une température dans la plage d'environ 0°C à environ 100°C ou dans la plage d'environ 0°C à environ 35°C ou dans la plage d'environ 10°C à environ 30°C.

8. Procédé selon l'une quelconque des revendications précédentes, dans lequel la précipitation comprend l'ajustement du pH à une valeur dans la plage d'environ 6 à environ 12 ou dans la plage d'environ 8 à environ 11.

9. Procédé selon l'une quelconque des revendications précédentes, dans lequel la solution acide comprend environ 5% à environ 50% d'acide et le cas échéant la solution acide est de l'acide acétique à environ 50% et la solution basique est de l'ammoniaque aqueux à environ 15%.

10. Procédé selon l'une quelconque des revendications précédentes, dans lequel la solution acide est de l'acide formique à environ 38% et la solution basique est de l'hydroxyde de sodium méthanolique à environ 10%, ou dans lequel la solution acide est de l'acide chlorhydrique à environ 10% et la solution basique est de l'hydroxyde de sodium aqueux à environ 10%, ou dans lequel la solution acide est de l'acide acétique à environ 43% et la solution basique est de l'ammoniaque aqueux à environ 25%, ou dans lequel la solution acide est de l'acide acétique à environ 40% et la solution basique est de l'hydroxyde de sodium aqueux à environ 50%, ou dans lequel la solution acide est de l'acide formique à environ 20% et la solution basique est de l'ammoniaque aqueux à environ 25%, ou dans lequel la solution acide est de l'acide acétique à environ 33% et la solution basique est de l'ammoniaque aqueux à environ 50%, ou dans lequel la solution acide est de l'acide acétique à environ 50% et la solution basique est de l'ammoniaque aqueux à environ 25%.

11. Forme polymorphe d'olanzapine de forme III ayant un spectre typique de diffraction des rayons X par poudre représenté par les écartements de plans suivants :
| Ecartements d(Å) |
|---|
| 10,3156 |
| 7,1713 |
| 6,5014 |
| 5,5165 |
| 4,8541 |
| 4,5578 |
| 4,4938 |
| 4,4536 |
| 4,2588 |
| 4,0699 |
| 3,9898 |
| 3,7288 |
| 3,5626 |
| 3,0262 |
le cas échéant caractérisée en plus en ce qu'elle présente sensiblement le spectre de diffraction des rayons X par poudre suivant, dans lequel d représenté l'écartement des plans et I/I₁ représente les intensités relatives typiques :
| Ecartement d (Å) | T/I₁ |
|---|---|
| 10,3156 | 100 |
| 7,1713 | 16 |
| 6,5014 | 17 |
| 5,5165 | 24 |
| 4,8541 | 46 |
| 4,5578 | 24 |
| 4,4938 | 38 |
| 4,4536 | 36 |
| 4,2588 | 49 |
| 3,9898 | 52 |
| 3,7288 | 42 |
| 3,5626 | 25 |
| 3,0262 | 18. |

12. Forme polymorphe d'olanzapine de forme III selon la revendication. 11, caractèrisée en plus en ce qu'elle possède un spectre infrarouge qui présente des absorbances aux nombres d'ondes suivants :
| |
|---|
| 611 |
| 656 |
| 671 |
| 746 |
| 765 |
| 845 |
| 935 |
| 1369. |

13. Forme polymorphe d'olanzapine de forme III selon la revendication 11 ou la revendication 12, obtenue par le procédé consistant soit à dissoudre de l'olanzapine de forme I ou de forme II dans de l'acide acétique aqueux à 50% et à précipiter de l'olanzapine de forme III pratiquement pure avec de l'ammoniaque aqueux à 15%, soit à dissoudre de l'olanzapine de forme I ou de forme II dans de l'acide acétique aqueux à environ 33% et à précipiter de l'olanzapine de forme III pratiquement pure avec de l'ammoniaque aqueux à environ 50%, l'olanzapine de forme I étant une forme polymorphe d'olanzapine ayant un spectre typique de diffraction des rayons X par poudre représenté par les écartements de plans suivants :
| Ecartements d (Å) |
|---|
| 9,9463 |
| 8,5579 |
| 8,2445 |
| 6,8862 |
| 6,3787 |
| 6,2439 |
| 5,5895 |
| 5,3055 |
| 4,9815 |
| 4,8333 |
| 4,7255 |
| 4,6286 |
| 4,533 |
| 4,4624 |
| 4,2915 |
| 4,2346 |
| 4,0855 |
| 3,8254 |
| 3,7489 |
| 3,6983 |
| 3,5817 |
| 3,5064 |
| 3,3392 |
| 3,2806 |
| 3,2138 |
| 3,1118 |
| 3,0507 |
| 2,948 |
| 2,8172 |
| 2,7589 |
| 2,6597 |
| 2,6336 |
| 2,5956 ; |
et l'olanzapine de forme II étant une forme polymorphe d'olanzapine ayant un spectre typique de diffraction des rayons X par poudre représenté par les écartements de plans suivants :
| Ecartements d (Å) |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516 |
| 3,134 |
| 3,0848 |
| 3,0638 |
| 3,0111 |
| 2,8739 |
| 2,8102 |
| 2,7217 |
| 2,6432 |
| 2,6007. |

14. Forme polymorphe d'olanzapine de forme IV ayant un spectre typique de diffraction des rayons X par poudre représenté par les écartements de plans suivants :
| Ecartements d (Å) |
|---|
| 9,9487 |
| 8,5074 |
| 8,2103 |
| 4,8172 |
| 4,7114 |
| 4,6122 |
| 4,5282 |
| 4,2340 |
| 4,0901 |
| 3,7574 |
| 3,6989. |
le cas échéant caractérisée en plus en ce qu'elle présente sensiblement le spectre de diffraction des rayons X par poudre suivant, dans lequel d représente l'écartement des plans et I/I₁ représente les intensités relatives typiques :
| Ecartement d (Å) | I/I₁ |
|---|---|
| 9,9487 | 83 |
| 8,5074 | 15 |
| 8,2103 | 17 |
| 4,8172 | 100 |
| 4,7114 | 41 |
| 4,6122 | 35 |
| 4,5282 | 33 |
| 4,2340 | 29 |
| 4,0901 | 32 |
| 3,7574 | 23 |
| 3,6989 | 40 |

15. Forme polymorphe d'olanzapine de forme IV selon la revendication 14, caractérisée en plus en ce qu'elle possède un spectre infrarouge qui présente des absorbances aux nombres d'ondes suivants :
| |
|---|
| 604 |
| 661 |
| 758 |
| 904 |
| 931 |
| 1365 |
| 1456. |

16. Forme polymorphe d'olanzapine de forme IV selon la revendication 14 ou la revendication 15, obtenue par le procédé consistant soit à dissoudre de l'olanzapine de forme I ou de forme II dans de l'acide formique aqueux à environ 38% et à précipiter de l'olanzapine de forme IV pratiquement pure en utilisant de l'hydroxyde de sodium méthanolique à environ 10%, soit à dissoudre de l'olanzapine de forme I ou de forme II dans de l'acide acétique aqueux à environ 43% et à précipiter de l'olanzapine de forme IV pratiquement pure en utilisant de l'ammoniaque aqueux à environ 25%, l'olanzapine de forme I étant une forme polymorphe d'olanzapine ayant un spectre typique de diffraction des rayons X par poudre représenté par les écartements de plans suivants :
| Ecartements d (Å) |
|---|
| 9,9463 |
| 8,5579 |
| 8,2445 |
| 6,8862 |
| 6,3787 |
| 6,2439 |
| 5,5895 |
| 5,3055 |
| 4,9815 |
| 4,8333 |
| 4,7255 |
| 4,6286 |
| 4,533 |
| 4,4624 |
| 4,2915 |
| 4,2346 |
| 4,0855 |
| 3,8254 |
| 3,7489 |
| 3,6983 |
| 3,5817 |
| 3,5064 |
| 3,3392 |
| 3,2806 |
| 3,2138 |
| 3,1118 |
| 3,0507 |
| 2,948 |
| 2,8172 |
| 2,7589 |
| 2,6597 |
| 2,6336 |
| 2,5956; |
et l'olanzapine de forme II étant une forme polymorphe d'olanzapine ayant un spectre typique de diffraction des rayons X par poudre représenté par les écartements de plans suivants :
| Ecartements d (Å) |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516. |
| 3,134 |
| 3,0848 |
| 3,0638 |
| 3,0111 |
| 2,8739 |
| 2,8102 |
| 2,7217 |
| 2,6432 |
| 2,6007. |

17. Forme polymorphe d'olanzapine de forme V ayant un spectre typique de diffraction des rayons X par poudre représenté par les écartements de plans suivants :
| Ecartements d (Å) |
|---|
| 10,5932 |
| 10,2170 |
| 9,9503 |
| 8,5259 |
| 7,1016 |
| 6,0731 |
| 5,2041 |
| 4,9856 |
| 4,8153 |
| 4,7514 |
| 4,5302. |
| 4,4714 |
| 4,2271 |
| 4,1307 |
| 3,9880 |
| 3,7763 |
| 3,7167 |
| 3,5315. |
le cas échéant caractérisée en plus en ce qu'elle présente sensiblement le spectre de diffraction des rayons X par poudre suivant, dans lequel d représente l'écartement des plans et I/I₁ représente les intensités relatives typiques :
| Ecartement d (Å) | I/I₁ |
|---|---|
| 10,5932 | 17 |
| 10,2170 | 1.00 |
| 9,9503 | 57 |
| 8,5259 | 22 |
| 7,1016 | 17 |
| 6,0731 | 17 |
| 5,2041 | 19 |
| 4,9856 | 20 |
| 4,8153 | 62 |
| 4,7514 | 34 |
| 4,5302 | 24 |
| 4,4714 | 51 |
| 4,2271 | 91 |
| 4,1307 | 40 |
| 3,9880 | 31 |
| 3,7763 | 10 |
| 3,7167 | 62 |
| 3,5315 | 22 |

18. Forme polymorphe d'olanzapine de fonne V selon la revendication 17, caractérisée en plus en ce qu'elle possède un spectre infrarouge qui présente des absorbances aux nombres d'ondes suivants :
| |
|---|
| 604 |
| 671 |
| 746 |
| 758 |
| 847 |
| 928 |
| 1357 |
| 1369. |

19. Forme polymorphe d'olanzapinc de forme V selon la revendication 17 ou la revendication 18, obtenue par le procédé consistant soit à dissoudre de l'olanzapine de forme I ou de forme II dans de l'acide chlorhydrique aqueux à environ 10% et à précipiter de l'olanzapine de forme V pratiquement pure en utilisant de l'hydroxyde de sodium aqueux à environ 10%, soit à dissoudre de l'olanzapine de forme I ou de forme II dans de l'acide acétique à environ 40% et à précipiter de l'olanzapine de forme V pratiquement pure en utilisant de l'hydroxyde de sodium aqueux à environ 50%, soit à dissoudre de l'olanzapine de forme I ou de forme II dans de l'acide fonnique aqueux à environ 20% et à précipiter de l'olanzapine de forme V pratiquement pure en utilisant de l'ammoniaque aqueux à environ 25%, soit à dissoudre de l'olanzapine de forme I ou de forme II dans de l'acide acétique aqueux à environ 50% et à précipiter de l'olanzapine de forme V pratiquement pure en utilisant de l'ammoniaque aqueux à environ 25%, l'olanzapine de forme I étant une forme polymorphe d'olanzapine ayant un spectre typique de diffraction des rayons X par poudre représenté par les écartements de plans suivants :
| Ecartements d (Å) |
|---|
| 9,9463 |
| 8,5579 |
| 8,2445 |
| 6,8862 |
| 6,3787 |
| 6,2439 |
| 5,5895 |
| 5,3055 |
| 4,9815 |
| 4,8333 |
| 4,7255 |
| 4,6286 |
| 4,533 |
| 4,4624 |
| 4,2915 |
| 4,2346 |
| 4,0855 |
| 3,8254 |
| 3,7489 |
| 3,6983 |
| 3,5817 |
| 3,5064 |
| 3,3392 |
| 3,2806 |
| 3,2138 |
| 3,1118 |
| 3,0507 |
| 2,948 |
| 2,8172 |
| 2,7589 |
| 2,6597 |
| 2,6336 |
| 2,5956 ; |
et l'olanzapine de forme II étant une forme polymorphe d'olanzapine ayant un spectre typique de diffraction des rayons X par poudre représenté par les écartements de plans suivants :
| Ecartements d (Å) |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516 |
| 3,134 |
| 3,0848 |
| 3,0638 |
| 3,0111 |
| 2,8739 |
| 2,8102 |
| 2,7217 |
| 2,6432 |
| 2,6007. |

20. Formulation pharmaceutique contenant comme principe actif au moins une forme polymorphe d'olanzapine associée à un ou plusieurs véhicules, excipients ou diluants pharmaceutiquement acceptables pour cette forme, dans laquelle la au moins une forme polymorphe d'olanzapine est de l'olanzapine de forme III, de l'olanzapine de forme IV ou de l'olanzapine de forme V, ainsi que des sels et des mélanges de celles-ci, et dans laquelle les formes III, IV et V de l'olanzapine sont des formes polymorphes d'olanzapine ayant des spectres typiques de diffraction des rayons X par poudre représentés par les écartements de plans suivants :
| FORME III | FORME IV | FORME V |
|---|---|---|
| Ecartement d (Å) | Ecartement d (Å) | Ecartement d (Å) |
| 10,3156 | 9,9487 | 10,5932 |
| 7,1713 | 8,5074 | 10,2170 |
| 6,5014 | 8,2103 | 9,9503 |
| 5,5165 | 4,8172 | 8,5259 |
| 4,8541 | 4,7114 | 7,1016 |
| 4,5578 | 4,6122 | 6,0731 |
| 4,4938 | 4,5282 | 5,2041 |
| 4,4536 | 4,2340 | 4,9856 |
| 4,2588 | 4,0901 | 4,8153 |
| 3,9898 | 3,7574 | 4,7514 |
| 3,7288 | 3,6989 | 4,5302 |
| 3,5626 | | 4,4714 |
| 3,0262 | | 4,2271 |
| | | 4,1307 |
| | | 3,9880 |
| | | 3,7763 |
| | | 3,7167 |
| | | 3,5315 |

21. Utilisation d'olanzapine de forme III, de forme IV ou de forme V selon l'une quelconque des revendications 11 à 20, ainsi que des sels et des mélanges de celles-ci, pour la fabrication d'un médicament destiné au traitement d'une condition psychotique, de l'anxiété modérée ou de conditions gastro-intestinales.
